# EUROPEAN PATENT SPECIFICATION

(11) **EP 2 765 171 B1**
(45) Date of publication and mention of the grant of the patent: **16.03.2016**
(21) Application number: 12838700.8
(22) Date of filing: 27.09.2012
(51) Int. Cl.: C09J 163/00, A61B 1/00, C09J 11/04, C09J 11/06, C09J 133/04, C09J 163/02, C09J 163/04, G02B 23/24, C08G 59/50

(54) **ADHESIVE COMPOSITION FOR MEDICAL APPARATUS AND ENDOSCOPIC DEVICE**
HAFTSTOFFZUSAMMENSETZUNG FÜR MEDIZINISCHE VORRICHTUNGEN UND ENDOSKOPISCHE VORRICHTUNGEN
COMPOSITION ADHÉSIVE POUR APPAREIL MÉDICAL ET DISPOSITIF ENDOSCOPIQUE

(30) Priority: 05.10.2011 JP 2011220837
(43) Date of publication of application: 13.08.2014
(73) Proprietor: Olympus Corporation, Shibuya-ku Tokyo 151-0072 (JP)
(72) Inventor: KOBAYASHI Koji, Tokyo 151-0072 (JP); NIINO Rieko, Tokyo 151-0072 (JP); NAKAMURA Mitsuhiro, Tokyo 151-0072 (JP); MATSUMOTO Jun, Tokyo 151-0072 (JP)
(74) Representative: Kuhnen & Wacker
(86) International application number: PCT/JP2012/074895
(87) International publication number: WO 2013/051458

(56) References cited:
- EP-A1- 1 437 393
- EP-A1- 1 847 213
- WO-A1-2011/126018
- JP-A- S6 448 877
- JP-A- 2002 238 834
- JP-A- 2002 338 662
- JP-A- 2005 152 461

## Description

### [Technical Field]

The present invention relates to an adhesive composition used in a medical instrument and an endoscope device.

Priority is claimed on Japanese Patent Application No. 2011-220837, filed October 5, 2011, the content of which is incorporated herein by reference.

### [Background Art]

A medical instrument such as an endoscope device or the like is generally configured such that a plurality of members are assembled using an adhesive.

The medical instrument generally passes through sterilization processing under a high temperature and high pressure vapor using an autoclave, or sterilization processing using chemicals such as peracetic acid, a gas (for example, hydrogen peroxide-based gas, ethylene oxide gas, or the like), or the like.

However, when the medical instrument is sterilized using the autoclave or the chemicals, the adhesive may be deteriorated by the saturated vapor or the chemicals and the members adhered to each other by the adhesive may be peeled off.

Here, as an endoscope in which deterioration in the vicinity of the adhesion parts cannot easily occur in spite of the sterilization processing, for example, in Patent Document 1, a technique of improving sterilization resistance by inserting a prescribed filler into the adhesive is disclosed.

In addition, as an endoscope having good air tightness and durability of a junction section between a lens and a frame body in spite of the sterilization processing, for example, in Patent Document 2, an endoscope including a lens junction body, in which the frame body and the lens are adhered by an adhesive including a thermosetting resin such as epoxy resin or the like, and a filler, is shown.

### [Prior Art Document]

### [Patent Document]

[Patent Document 1] Japanese Unexamined Patent Application, First Publication No. 2004-358006
[Patent Document 2] Japanese Unexamined Patent Application, First Publication No. 2005-234239

### [Summary of the Invention]

### [Problems to be Solved by the Invention]

However, the adhesive used in the endoscope disclosed in Patent Document 1 does not have sufficient resistance with respect to a plurality of times of sterilization processing. For this reason, when the sterilization processing is repeated, the members adhered by the adhesive may be peeled off. In addition, when the sterilization processing is performed, the adhesive may be discolored, cracked, or dissolved, and the appearance of an adhesive layer of an outer surface of the endoscope may be damaged.

In the endoscope disclosed in Patent Document 2, resistance of the adhesive with respect to the sterilization processing is insufficient. For this reason, an adhesive strength of the adhesive may be easily decreased, and the members adhered by the adhesive may be peeled off. In addition, when the sterilization processing is performed, the adhesive may be discolored, cracked, or dissolved, and the appearance of an adhesive layer of an outer surface of the endoscope may be damaged.

In consideration of the above-mentioned circumstances, the present invention is directed to provide an adhesive composition used in a medical instrument having good sterilization resistance even when sterilization processing is repeated and capable of appropriately maintaining an adhesive strength or appearance, and an endoscope device configured such that appearance of an adhesive composition is good even when sterilization processing is repeated and members adhered by the adhesive composition cannot be easily peeled off.

### [Means for Solving the Problems]

An adhesive composition used in a medical instrument according to a first aspect of the present invention is an adhesive composition used in a medical instrument including a main agent that includes one or more kinds of epoxy resin selected from the group consisting of bisphenol A-type epoxy resins, bisphenol F-type epoxy resins, and phenol novolac type epoxy resins; a curing agent containing meta xylylene diamine and/or a derivative thereof; acrylic rubber; and a filler which contains alumina.

According to a second aspect of the present invention, in the adhesive composition used in a medical instrument according to the first aspect, the filler may include at least plate-like alumina having an aspect ratio of 2 to 99.

According to a third aspect of the present invention, in the adhesive composition used in a medical instrument according to the second aspect, the filler may further include spherical alumina having an average particle diameter of 1 to 100 µm, and amorphous shape alumina having an average particle diameter of 1 to 500 nm.

According to a fourth aspect of the present invention, in the adhesive composition used in a medical instrument according to the first aspect, the filler may include one or more kinds of alumina selected from the group consisting of plate-like alumina having an aspect ratio of 2 to 99, spherical alumina having an average particle diameter of 1 to 100 µm, and amorphous shape alumina having an average particle diameter of 1 to 500 nm. When a total content of the main agent, the curing agent and the acrylic rubber is 100 parts by mass, a content of the plate-like alumina may be 10 to 150 parts by mass when the filler includes the plate-like alumina, a content of the spherical alumina may be 10 to 150 parts by mass when the filler includes the spherical alumina, and a content of the amorphous shape alumina may be 1 to 20 parts by mass when the filler includes the amorphous shape alumina, and wherein when the filler includes all of the plate-like alumina, the spherical alumina and the amorphous shape alumina, a sum of a content of the plate-like alumina, the spherical alumina and the amorphous shape alumina may be 10 to 150 weight parts by mass

According to a fifth aspect of the present invention, an endoscope device is assembled by using the adhesive composition according to any one of first to fourth aspect of the present invention.

### [Advantageous Effects of Invention]

The adhesive composition used in a medical instrument has good sterilization resistance even when sterilization processing is repeated, and can appropriately maintain an adhesive strength or visual appearance.

In the endoscope device, even when the sterilization processing is repeated, the visual appearance of the adhesive composition can be appropriately maintained, and the members adhered by the adhesive composition cannot be easily peeled off.

### [Brief Description of Drawings]

Fig. 1 is a schematic view schematically showing an example of a cured material formed by hardening an adhesive composition used in a medical instrument according to an embodiment of the present invention.
Fig. 2 is a perspective view showing an example of a schematic configuration of an endoscope device assembled by using the adhesive composition used in a medical instrument according to the embodiment of the present invention.
Fig. 3 is a front view of a distal end section of an endoscope device shown in Fig. 2.
Fig. 4 is a cross-sectional view taken along line A-A of Fig. 3.

### [Best Modes for Carrying out the Invention]

Hereinafter, an embodiment of the present invention will be described in detail.

### [Adhesive composition used in a medical instrument]

An adhesive composition used in a medical instrument according to the embodiment (hereinafter, simply referred to as an "adhesive composition") is a two-liquid type adhesive composition containing a main agent, a curing agent, acrylic rubber, and a filler, and a chemical reaction is performed by heating to accelerate curing.

### <Main agent>

The main agent that includes one or more kinds of epoxy resin selected from the group consisting of bisphenol A-type epoxy resins, bisphenol F-type epoxy resins, and phenol novolac type epoxy resins. The main agent preferably contains three kinds of epoxy resins of the bisphenol A-type epoxy resins, the bisphenol F-type epoxy resins, and the phenol novolac type epoxy resins in order to improve sterilization resistance when sterilization processing is repeated and obtain an appropriate viscosity or a high adhesive strength.

### <Curing agent>

The curing agent includes one or both meta xylylene diamine and derivatives thereof. In particular, the curing agent preferably include a meta xylylene diamine and derivatives thereof in order to increase a reaction velocity with the main agent.

As the derivatives of the meta xylylene diamine, for example, an alkylene oxide additive, glycidyl ester additive, glycidyl ether additive, Mannich additive, acrylonitrile additive, epichlorodydrin additive, xylylene diamine trimer, and so on, may be provided.

In addition, in description of the embodiment, the meta xylylene diamine and derivatives thereof may be referred to as an "amine-based curing agent."

The amount of the meta xylylene diamine is preferably 1 to 90 mass% and is more preferably 3 to 70 mass% when the total mass of the curing agent is 100 mass%. When the amount of the meta xylylene diamine is within the above ranges, an appropriate reaction velocity can be obtained and suppression of the reaction with carbonic acid gas in air or improvement in adhesive strength can be obtained.

The amount of the derivative of the meta xylylene diamine is preferably 10 to 95 mass%, is more preferably 30 to 90 mass% when the total weight of the curing agent is 100 mass%. When the amount of the derivative of the meta xylylene diamine is within this range, an appropriate reaction velocity can be obtained and suppression of the reaction with carbonic acid gas in air or improvement in adhesive strength can be obtained.

The curing agent may further include another compound other than the amine-based curing agent. As the other compound, for example, polyamide resins, imidazoles, acid anhydrides, and so on, may be provided.

A compounding ratio of the main agent and the curing agent may be set to cause a reaction between an epoxy group of the epoxy resin, which is the main agent, and a functional group of the curing agent reacted with the epoxy group at a chemical equivalent.

Here, in the main agent of the epoxy resin, a molecular weight per one functional group is referred to as an epoxy equivalent, and an amine equivalent of the amine-based curing agent is referred to as an active hydrogen equivalent. A theoretical compounding ratio is calculated from the epoxy equivalent and the amine equivalent to be set as a guideline of an appropriate compounding ratio, and further, an optimal compounding ratio of the main agent and the curing agent is set from the adhesive strength or the like.

In consideration of the above-mentioned description, the compounding ratio (a mass ratio) of the main agent and the curing agent is preferably 10:1 to 10:9, more preferably 10:1 to 10:7. When the compounding ratio of the main agent and the curing agent is within the above ranges, oxidation degradation, hydrolysis, softening deterioration by heat, hardening deterioration, brittle fracture and reduction in adhesive strength can be suppressed.

### <Acrylic rubber>

The acrylic rubber serves to increase moisture resistance and heat resistance against the sterilization processing, in particular, the sterilization processing under the high temperature and high pressure vapor, to the adhesive composition. Thereby the adhesive composition can appropriately maintain the adhesive strength.

The acrylic rubber is preferably a fine powder having a mean grain diameter of 300 nm or less, and is preferably dispersed in the epoxy resin that constitutes the main agent. The reasons for this are as follows.

The adhesive including the bisphenol type or phenol novolac type epoxy resin and the acrylic rubber forms a sea-island structure in which the acrylic rubber is dispersed in the epoxy resin like islands when the adhesive is heated for the curing reaction, and the adhesive property such as high temperature and high humidity sterilization resistance or the like can be easily exhibited. In general, while formation of the sea-island structure tends to depend on the blending condition of the epoxy resin and the acrylic rubber or the curing condition, when the acrylic rubber is dispersed in the epoxy resin, the sea-island structure can be easily formed substantially regardless of the blending condition or the curing condition. As a result, the degree of freedom of an adhesion operation, the curing condition, or the like, can be increased.

The amount of the acrylic rubber is preferably 1 to 20 mass%, and is more preferably 5 to 15 mass% when the sum of the amounts of the main agent and the acrylic rubber is 100 mass%. When the amount of the acrylic rubber is within the above ranges, an adhesive composition having good adhesion shearing strength and adhesion separation strength can be obtained. In addition, the crosslink density is increased, and the moisture and heat resistance, and the chemical resistance of the cured material of the adhesive composition can be improved. Accordingly, the adhesive composition that can exhibit a sufficient adhesive strength in spite of the sterilization processing under the high temperature and high pressure vapor or the sterilization processing using the chemicals can be easily obtained.

### <Filler>

The adhesive composition according to the embodiment includes alumina as a filler. The alumina provides a chemical resistance against the sterilization processing using the chemicals to the adhesive composition, and appropriately maintains the adhesive strength or a visual appearance.

As the alumina, plate-like alumina, spherical alumina, amorphous shape alumina, or the like, are appropriate. Among these, the plate-like alumina is preferably used at least in order to improve the chemical resistance, and combined use of the plate-like alumina, the spherical alumina and the amorphous shape alumina is particularly preferable.

The reasons that the filler preferably includes at least the plate-like alumina, and further, in particular, preferably includes the spherical alumina and the amorphous shape alumina, are considered to be as follows.

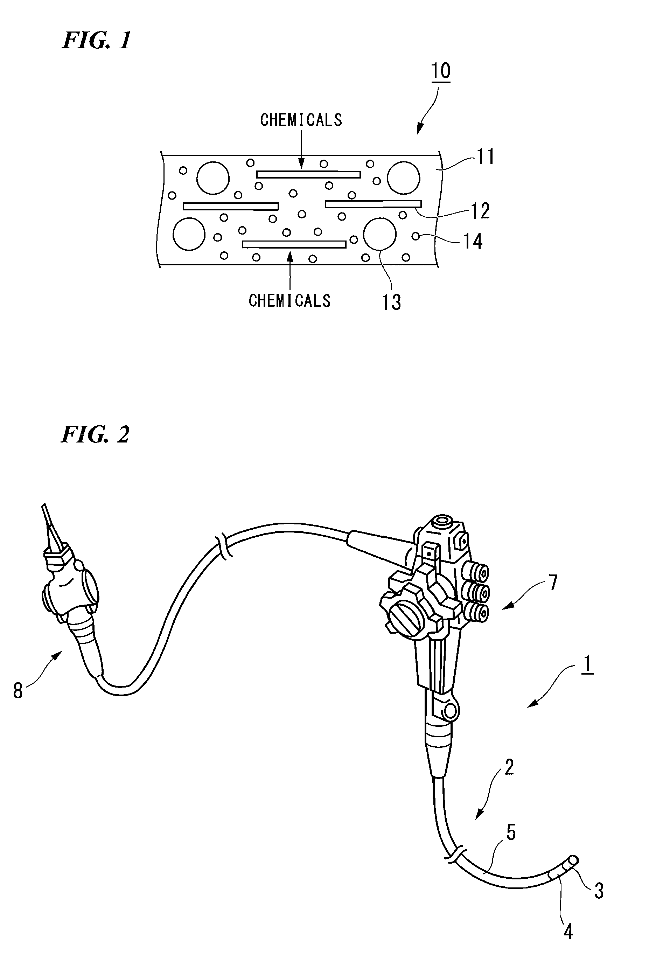

When the filler includes the plate-like alumina and the adhesive composition is cured, as shown in Fig. 1, a cured material (an adhesive layer) 10 in which plate-like alumina 12 as the filler is dispersed in a resin section 11 which is formed by the curing reaction between the main agent, the acrylic rubber and the curing agent is obtained. Accordingly, even when the cured material 10 is exposed to the chemicals such as peracetic acid or the like, penetration of the chemicals into the resin section 11 can be effectively prevented by the plate-like alumina 12, and the chemical resistance against the sterilization processing using the chemicals can be improved, appropriately maintaining the adhesive strength or visual appearance.

In addition, when the filler further includes spherical alumina and amorphous shape alumina in addition to the plate-like alumina, as shown in Fig. 1, spherical alumina 13 or amorphous shape alumina 14 is dispersed between the plate-like alumina 12 and the plate-like alumina 12. Accordingly, since a packing density of the filler is increased, penetration of the chemicals into the resin section 11 can be more effectively prevented.

In Fig. 1, for the convenience of illustration, the amorphous shape alumina 14 is represented as circles (spherical shapes).

In the embodiment, the "plate shape" means that an aspect ratio between the largest diameter and the thickness of the alumina (largest diameter/thickness) is 2 to 99. The aspect ratio of the plate-like alumina is more preferably 5 to 50 in that the sterilization resistance can be improved and the visual appearance can be also appropriately maintained even when the sterilization processing is repeated.

In addition, the average particle diameter of the plate-like alumina is preferably 0.05 to 20 µm, and more preferably 0.1 to 10 µm, in particular, preferably 0.4 to 4 µm in order to improve the sterilization resistance and appropriately maintain the visual appearance even when the sterilization processing is repeated.

The average particle diameter of the plate-like alumina is a value measured by an X-ray transmission type particle size distribution measurement apparatus.

In addition, the "spherical alumina" is a particle in which the particle shape is a spherical shape when observed by an electron microscope.

The average particle diameter of the spherical alumina is preferably 1 to 100 µm, and more preferably 2 to 50 µm in order to improve working property or sterilization resistance when the adhesive composition is applied and appropriately maintain the visual appearance even when the sterilization processing is repeated.

The average particle diameter of the spherical alumina is a 50% average value of a median size of a volume average particle diameter, which is measured by a particle distribution measurement apparatus using a laser diffraction scattering method.

In addition, the "amorphous shape alumina" is particles in which the particle shape when observed by an electron microscope is a spherical shape, a needle shape, a plate shape, an oval shape, a polygonal shape, or the like, not limited to a specific shape.

The average particle diameter of the amorphous shape alumina is preferably 1 to 500 nm, and is more preferably 10 to 100 nm in order to improve the working property or the sterilization resistance and more appropriately maintain the visual appearance even when the sterilization processing is repeated.

The average particle diameter of the amorphous shape alumina is a value measured by the X-ray penetration type particle size distribution measurement apparatus.

The amount of the filler is as follows when a total amount of the main agent, the curing agent and the acrylic rubber is 100 parts by mass.

When the filler includes the plate-like alumina, the amount of the plate-like alumina is preferably 10 to 150 parts by mass, more preferably 10 to 80 parts by mass. When the amount of the plate-like alumina is within the above ranges, the chemical resistance can be improved and the adhesive strength or visual appearance can be appropriately maintained. In particular, when the amount is 80 parts by mass or less, a working property can be appropriately maintained.

When the filler includes the spherical alumina, the amount of the spherical alumina is preferably 10 to 150 parts by mass, more preferably 10 to 80 parts by mass, and in particular, preferably 30 to 60 parts by mass. When the amount of the spherical alumina is within the above ranges, the chemical resistance can be improved and the adhesive strength or visual appearance can be appropriately maintained. In particular, when the amount is 80 parts by mass or less, the working property can be appropriately maintained.

When the filler includes the amorphous shape alumina, the amount of the amorphous shape alumina is preferably 1 to 20 parts by mass, more preferably 1 to 8 parts by mass, and in particular, most preferably 2 to 6 parts by mass. When the amount of the amorphous shape alumina is within the above ranges, the chemical resistance can be improved and the adhesive strength or visual appearance can be appropriately maintained. In particular, when the amount is 8 parts by mass or less, the working property can be appropriately maintained.

When the filler includes the plate-like alumina, the spherical alumina and the amorphous shape alumina, a total of these amounts may be 10 to 150 parts by mass, and is more preferably 20 to 80 parts by mass. When the sum of the amounts is within the above ranges, the chemical resistance can be improved and the adhesive strength or visual appearance can be appropriately maintained. In particular, when the sum is 80 parts by mass or less, the working property can be appropriately maintained.

### <Other ingredients>

The adhesive composition according to the embodiment may include additives used for the conventional adhesive, for example, a catalyst, adhesive-providing agent, solvent, plasticizer, antioxidant, polymerization inhibitor, surfactant, anti-mold agent, coloring agent, and so on, according to necessity, as long as the effect of the present invention is not damaged, in addition to the above-mentioned main agent, curing agent, acrylic rubber, and filler.

### <Effects>

Since the adhesive composition according to the above-mentioned embodiment contains acrylic rubber and alumina as filler, in addition to the main agent and the curing agent, good sterilization resistance is provided even when the sterilization processing is repeated. Accordingly, for example, the adhesive strength or visual appearance can be appropriately maintained even under a saturated vapor of about 137 °C and 23300 hPa, under a peracetic acid water environment, under a gas environment such as hydrogen peroxide-based gas, or the like.

Accordingly, the adhesive composition according to the embodiment is appropriate for an adhesive used to assemble of the medical instrument, i.e., bonding members that constitute a medical instrument on which the sterilization processing is performed, i.e., the adhesive used to attach the members that constitute the medical instrument.

As the sterilization processing, sterilization processing under a high temperature and high pressure vapor (for example, autoclave sterilization), sterilization processing using chemicals, or the like, may be provided. Further, as the sterilization processing using chemicals, immersion sterilization including immersion in chemicals such as peracetic acid or the like, gas sterilization using a gas (for example, low temperature plasma sterilization using hydrogen peroxide-based gas, sterilization using ethylene oxide gas, sterilization using peracetic acid-based gas, or the like) may be provided.

### <Used method>

For example, adhesion of the members of the endoscope device using the adhesive composition according to the embodiment is performed as follows.

First, an "A" liquid including the main agent and a "B" liquid including the curing agent are mixed at a predetermined ratio. A predetermined amount of acrylic rubber and a filler, and additives according to necessity, are added to the mixed liquid. Next, the obtained mixture is applied to predetermined surfaces of the members of the endoscope device, and the surfaces are adhered and fixed. After that, as the members are heated at a predetermined temperature for a predetermined time, the members of the endoscope device are strongly adhered.

According to the same technique, sealing of the imaging device of the endoscope device, and exterior surfacing and fixing of a distal end of a flexible outer tube can be performed. Further, piling up of the adhesive layer on surroundings of the observing lens or illuminating lens can also be performed by the same technique.

The acrylic rubber, the filler, and the additives may be added to the main agent beforehand.

Further, the acrylic rubber may be dispersed in the bisphenol type or phenol novolac type epoxy resin.

While the heating temperature differs according to the kinds of the main agent and the curing agent included in the adhesive composition, the compounding ratio, or the like, the temperature is preferably 60 °C to 135 °C. In addition, the heating time may be about 0.5 to 3 hours.

When the heating temperature is less than 60 °C, progress of the curing reaction is delayed, and curing is time-consuming. When the heating temperature is higher than 135 °C, the endoscope device member having low thermal resistance may cause thermal degradation.

The members adhered using the adhesive composition of the embodiment is not limited thereto. For example, end-opening sections of various tubes inserted into an insertion unit of the endoscope device can be fixed to a distal end of the insertion unit or a manipulation unit by using the adhesive composition of the embodiment. In addition, a lens group disposed at a distal end hard section of the insertion unit can also be fixed to a lens frame or the distal end hard section. Further, a fiber bundle passing through the insertion unit can be fixed to the lens frame or the distal end hard section, or a charge coupled device (CCD) or the like inserted into the distal end hard section can be protected or fixed.

In addition, after a distal end of a flexible outer tube of the insertion unit of the endoscope device is tied from the outside by a thread and fixed to an inner member, the adhesive composition may be applied to the thread. When the adhesive composition is applied as described above, securing of an insertion easiness by the exterior surfacing and prevention of raveling of the thread can be realized at the same time.

### [Endoscope device]

Hereinafter, an endoscope device assembled using the adhesive composition according to the embodiment will be described with reference to Figs. 2 to 4.

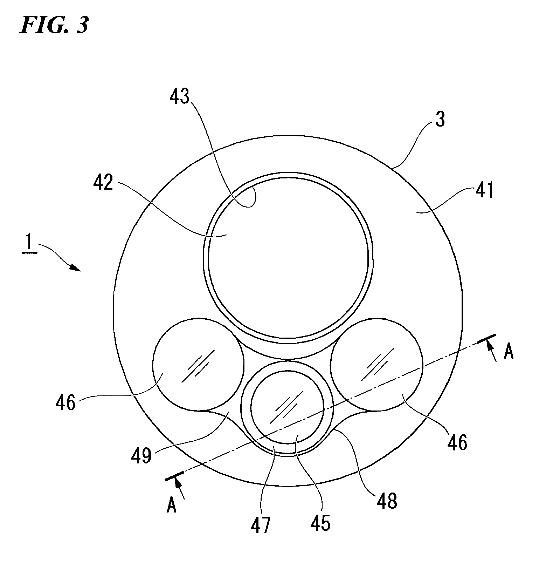

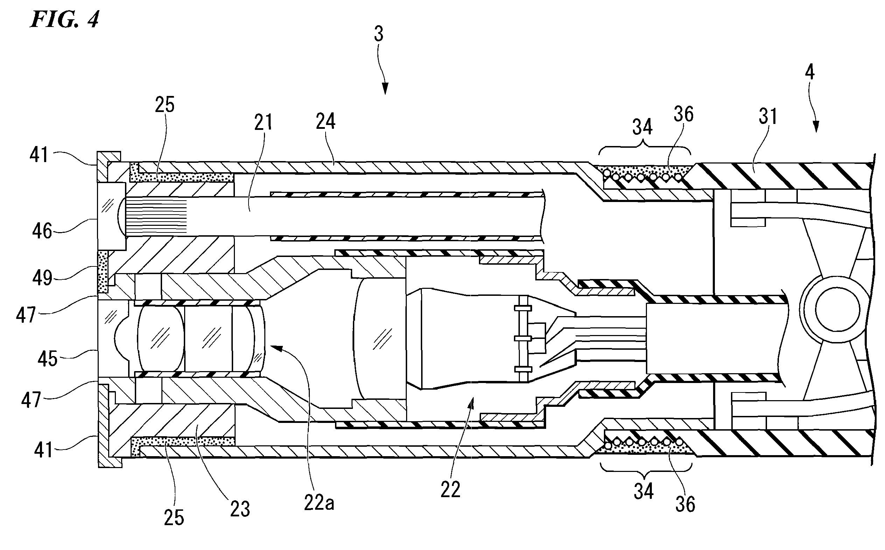

Fig. 2 is a perspective view showing an example of a schematic structure of the endoscope device of the embodiment, Fig. 3 is a front view of a distal end section of the endoscope device shown in Fig. 2, and Fig. 4 is a cross-sectional view taken along line A-A of Fig. 3.

In addition, in Fig. 4, the same components as in Fig. 3 are designated by the same reference numerals, and a description thereof will be omitted here.

As shown in Fig. 2, a schematic configuration of an endoscope device 1 of the embodiment is comprised with an elongated insertion unit 2 to be inserted into the body of a subject, a manipulation unit 7 connected to the insertion unit 2, and a universal code 8 electrically connected to the manipulation unit 7 and configured to supply illumination light.

A distal end section 3 configured to radiate illumination light from the distal end and receive the reflected light from the body, and a bendable section 4 and a flexible tube 5 configured to accommodate an optical fiber configured to transmit light received by the distal end section 3 and have bendable shapes are installed at the distal end side of the insertion unit 2.

In the above-mentioned endoscope device 1, the members adhered using the adhesive composition according to the embodiment are not particularly limited to specific ones as long as the members are components of the endoscope device 1. Hereinafter, a use state in the embodiment will be exemplarily described.

The adhesive composition according to the embodiment may be disposed, for example, around a lens frame of the distal end section 3 of the endoscope device 1.

Fig. 3 is a front view of the distal end section 3 of the endoscope device 1. A forceps channel 42 is provided at an insulating member 41, and a forceps cap 43 is disposed at a distal end section of the forceps channel 42. An object lens 45 installed in an object lens frame 47 is disposed between two illumination lenses 46, the adhesive composition of the embodiment is filled in a space between the illumination lenses 46 and the object lens frame 47, and a partition wall 48 is formed by a cured material 49 of the adhesive composition. Accordingly, incidence of direct light from the illumination lens 46 into the object lens 45 is prevented, and the illumination lens 46 and the object lens frame 47 are fixed by the cured material 49.

In addition, as shown in Fig. 4, a light guide fiber 21 configured to supply illumination light and a distal end hard section 23 having a columnar block shape and configured to hold an imaging unit 22 are installed at the distal end section 3 of the endoscope device 1, and a distal end cover 24 is fitted onto a side surface of the distal end hard section 23. In the embodiment, an adhesive layer 25 in which the adhesive composition according to the embodiment is filled is provided at a fitting part of the distal end hard section 23 and the distal end cover 24, and the distal end hard section 23 and the distal end cover 24 are adhered together.

Further, a tubular bendable rubber 31 configured to cover an outer circumference of the bendable section 4 is fitted onto a proximal end side of the distal end cover 24 from outside, a thread is wound around the bendable rubber 31 at the fitted portion of the bendable rubber 31 to form a spool section 34, and thus the bendable rubber 31 is fixed to the distal end cover 24. Next, an adhesive layer 36 formed by applying the adhesive composition according to the embodiment is formed on the outer circumference of the spool section 34, and securing of the insertion easiness by the exterior surfacing and prevention of raveling of the thread can be simultaneously realized. That is, the adhesive layer 36 is configured to cover the spool section 34 along the distal end cover 24 and a side surface of the bendable rubber 31, and the distal end section 3 and the bendable section 4 come in smooth contact with a living body to be slidable therewith when inserting the insertion unit 2.

Furthermore, in the endoscope device 1, end-opening sections of various tubes inserted into the insertion unit 2 of the endoscope device 1 can be fixed to the distal end of the insertion unit 2 or the manipulation unit 7 using the adhesive composition according to the embodiment. In addition, in the endoscope device 1, a lens group 22a or the like disposed at the distal end hard section 23 of the insertion unit 2 can be fixed to a lens frame or the distal end hard section 23 using the adhesive composition according to the embodiment. Further, a fiber bundle inserted into the insertion unit 2 may be fixed to the lens frame or the distal end hard section 23. Further, the CCD or the like of the imaging unit 22 inserted into the distal end section 3 can be protected, fixed and sealed.

In addition, while not shown in Fig. 2, an outer circumference of a connecting section between the bendable section 4 and the flexible tube 5 has the same configuration as the outer circumference of the connecting section between the distal end section 3 and the bendable section 4. Specifically, a spool section (not shown) is formed at the connecting section between the bendable section 4 and the flexible tube 5, and the adhesive composition according to the embodiment is applied on an outer circumference of the spool section. Securing of the insertion easiness by smoothly finishing the outer surface and prevention of raveling of the thread can be realized at the same time by the adhesive layer (not shown) formed by curing the adhesive composition.

Further, corner sections of a lens outer circumference can be smoothly surfaced through sealing of the imaging element of the endoscope device, or heaping the adhesive composition around an observing lens or an illuminating lens of the endoscope device.

The endoscope device according to the above-mentioned embodiment performs adhesion of the members of the endoscope device using the adhesive composition of the above-mentioned embodiment, adhering of the members of the endoscope device, exterior surfacing of the insertion unit of the endoscope device with respect to the flexible outer tube end section and fixing of the thread, sealing of the imaging element of the endoscope device, serving the adhesive composition around the observing lens or the illuminating lens of the endoscope device and smoothing of the corners of the lens outer circumference, or the like. Accordingly, even when the sterilization processing is repeated, since the visual appearance of the adhesive composition is kept good and good adhesive strength in the junction section or the like can be maintained, the members adhered together by the adhesive composition cannot be easily separated.

In addition, since the endoscope device according to the embodiment has no necessity of applying powder having a chemical resistance to a surface of the adhesive after supplying the adhesive to a section to be adhered, and forming a powder diffusion layer in the vicinity of the surface of the adhesive layer, as disclosed in Patent Document 1, a working process can be reduced and labor amount can be reduced.

### [Example]

Hereinafter, while the present invention has been specifically described with respect to an example, the present invention is not limited thereto.

### <Preparation of composition>

### (Composition A)

A bisphenol A-type epoxy resin of 30 parts by mass, a bisphenol F-type epoxy resin of 40 parts by mass, and a phenol novolac type epoxy resin of 30 parts by mass were mixed to prepare the main agent. Acrylic rubber of 20 parts by mass and a meta xylylene diamine derivative of 40 parts by mass were mixed with the main agent as the curing agent to prepare a composition A.

### (Compositions B to D)

Compositions B to D were prepared like the composition A except that the formulation was varied as shown in Table 1.

In addition, in the composition D, in addition to the acrylic rubber and the curing agent, silica was mixed with the main agent.

**[Table 1]**

| Composition | | | A | B | C | D |
|---|---|---|---|---|---|---|
| Mixing of composition (parts by mass) | Main agent | Bisphenol A type epoxy resin | 30 | 65 | 45 | 65 |
| | | Bisphenol F type epoxy resin | 40 | 0 | 55 | 0 |
| | | Phenol novolac type epoxy resin | 30 | 35 | 0 | 35 |
| | Curing agent | Meta xylylene diamine derivative | 40 | 40 | 40 | 40 |
| | Acrylic rubber | | 20 | 20 | 20 | 20 |
| | Silica | | 0 | 0 | 0 | 40 |

### [Example 1]

### <Preparation of adhesive composition>

The composition A of 100 parts by mass and the plate-like alumina (average particle diameter: 0.6 µm, aspect ratio: 10) of 10 parts by mass as the filler were mixed to prepare the adhesive composition.

Formulations of the obtained adhesive composition are represented in Table 2.

In addition, the obtained adhesive composition was measured and estimated as follows. The result is represented in Table 3.

### <Estimation>

### (1) Measurement of initial adhesive strength

A flat plate formed of stainless steel (SUS) and an engineering plastic flat plate formed of polysulfone resin (PSF) were adhered using the obtained adhesive composition through curing at 80 °C for two hours and used as a specimen.

An adhesive strength test on the obtained specimen was performed and adhesive strength was measured. In addition, the adhesive strength test was performed pursuant to JIS K6850 [Tensile shearing adhesion strength test method of adhesive].

### (2) Measurement of adhesive strength after sterilization processing

A specimen was made as in Estimation (1).

The obtained specimen was processed through gas sterilization by a low temperature plasma sterilization apparatus using hydrogen peroxide-based gas. After the specimen passed through the gas sterilization processing was extracted to the atmosphere, the specimen was processed through gas sterilization by the low temperature plasma sterilization apparatus using hydrogen peroxide-based gas again. With respect to the specimen, after the manipulation was repeated a total of 100 times, the adhesive strength test was performed as in Estimation (1), and the adhesive strength was measured.

### (3) Appearance estimation

The adhesive composition was applied on the flat plate formed of SUS, and cured at 80 °C for two hours to manufacture an appearance estimation specimen 1 in which an adhesive layer (a film thickness of 100 µm) was formed on the flat plate.

The adhesive composition was separately applied on the engineering plastic flat plate, and cured at 80 °C for two hours to manufacture an appearance estimation specimen 2 in which an adhesive layer (a film thickness of 100 µm) was formed on the engineering plastic flat plate.

Gas sterilization processing of the obtained appearance estimation specimens 1 and 2 was repeated as in Estimation (2) 100 times. The adhesive layers of the appearance estimation specimens 1 and 2 after the gas sterilization processing were observed by the naked eye and estimated according to the following estimation standard.
⊚: In both of the appearance estimation specimens 1 and 2, the adhesive layers did not change.
○: While the adhesive layer of at least one of the appearance estimation specimens 1 and 2 was discolored, there was no exfoliation.
Δ: The adhesive layer of at least one of the appearance estimation specimens 1 and 2 was slightly exfoliated.
×: The adhesive layer of at least one of the appearance estimation specimens 1 and 2 was exfoliated, cracked or dissolved.

### (4) Estimation of working property

A working property when the adhesive composition is applied on the flat plate formed of SUS was estimated pursuant of the following estimation standard.
○: Easy application is possible.
Δ: Application is slightly difficult, but there is no problem in use.
×: Application is difficult.

### [Examples 2 to 32]

The adhesive composition was prepared as in Example 1 except that kinds and mixing of alumina were varied as represented in Table 2, and measurement and estimation was performed. The result is represented in Table 3.

### [Comparative examples 1 to 4]

Measurement and estimation were performed as in Example 1 except that the kinds of compositions represented in Table 2 were used as the adhesive composition. The result is represented in Table 3.

**[Table 2]**

| | Kind of composition | Plate-like alumina | | | Spherical alumina | | Amorphous shape alumina | |
|---|---|---|---|---|---|---|---|---|
| | | Average particle diameter (µm) | Aspect ratio | Amount (parts by mass) | Average particle diameter (µm) | Amount (parts by mass | Average particle diameter (nm) | Amount (parts by mass) |
| Example 1 | A | 0.6 | 10 | 10 | - | - | - | - |
| Example 2 | A | 0.6 | 10 | 5 | - | - | - | - |
| Example 3 | A | 0.6 | 10 | 40 | - | - | - | - |
| Example 4 | A | 0.6 | 10 | 80 | - | - | - | - |
| Example 5 | A | 0.6 | 10 | 100 | - | - | - | - |
| Example 6 | A | 0.1 | 10 | 40 | - | - | - | - |
| Example 7 | A | 0.05 | 10 | 40 | - | - | - | - |
| Example 8 | A | 10 | 10 | 40 | - | - | - | - |
| Example 9 | A | 20 | 10 | 40 | - | - | - | - |
| Example 10 | A | 0.6 | 50 | 40 | - | - | - | - |
| Example 11 | A | 0.6 | 70 | 40 | - | - | - | - |
| Example 12 | A | 0.6 | 5 | 40 | - | - | - | - |
| Example 13 | A | 0.6 | 2 | 40 | - | - | - | - |
| Example 14 | A | - | - | - | 10 | 40 | - | - |
| Example 15 | A | - | - | - | 10 | 80 | - | - |
| Example 16 | A | - | - | - | 10 | 100 | - | - |
| Example 17 | A | - | - | - | 10 | 5 | - | - |
| Example 18 | A | - | - | - | 10 | 10 | - | - |
| Example 19 | A | - | - | - | 1 | 40 | - | - |
| Example 20 | A | - | - | - | 2 | 40 | - | - |
| Example 21 | A | - | - | - | 50 | 40 | - | - |
| Example 22 | A | - | - | - | 60 | 40 | - | - |
| Example 23 | A | - | - | - | - | - | 50 | 5 |
| Example 24 | A | - | - | - | - | - | 50 | 0.5 |
| Example 25 | A | - | - | - | - | - | 50 | 1 |
| Example 26 | A | - | - | - | - | - | 50 | 8 |
| Example 27 | A | - | - | - | - | - | 50 | 10 |
| Example 28 | A | - | - | - | - | - | 5 | 5 |
| Example 29 | A | - | - | - | - | - | 10 | 5 |
| Example 30 | A | - | - | - | - | - | 100 | 5 |
| Example 31 | A | - | - | - | - | - | 150 | 5 |
| Example 32 | A | 0.6 | 10 | 40 | 10 | 30 | 50 | 5 |
| Comparative Example 1 | B | - | - | - | - | - | - | - |
| Comparative Example 2 | C | - | - | - | - | - | - | - |
| Comparative Example 3 | A | - | - | - | - | - | - | - |
| Comparative Example 4 | D | - | - | - | - | - | - | - |

**[Table 3]**

| | Adhesion strength (MPa) | | Appearance estimation | Estimation of work property |
|---|---|---|---|---|
| | Initial | After sterilization | | |
| Example 1 | 20 | 4 | ⊚ | ○ |
| Example 2 | 20 | 4 | Δ | ○ |
| Example 3 | 19 | 13 | ⊚ | ○ |
| Example 4 | 19 | 7 | ⊚ | Δ |
| Example 5 | 19 | 7 | ⊚ | × |
| Example 6 | 21 | 4 | ○ | Δ |
| Example 7 | 20 | 8 | Δ | ○ |
| Example 8 | 21 | 4 | ○ | Δ |
| Example 9 | 18 | 6 | Δ | ○ |
| Example 10 | 18 | 7 | ○ | ○ |
| Example 11 | 18 | 8 | Δ | ○ |
| Example 12 | 17 | 6 | ○ | ○ |
| Example 13 | 17 | 6 | Δ | ○ |
| Example 14 | 20 | 11 | ⊚ | ○ |
| Example 15 | 20 | 12 | ⊚ | Δ |
| Example 16 | 17 | 7 | ⊚ | × |
| Example 17 | 17 | 7 | Δ | ○ |
| Example 18 | 21 | 3 | ○ | ○ |
| Example 19 | 21 | 6 | ⊚ | × |
| Example 20 | 19 | 7 | ○ | ○ |
| Example 21 | 23 | 6 | ○ | ○ |
| Example 22 | 23 | 6 | Δ | ○ |
| Example 23 | 20 | 9 | ⊚ | ○ |
| Example 24 | 21 | 6 | Δ | ○ |
| Example 25 | 21 | 6 | ○ | ○ |
| Example 26 | 16 | 8 | ⊚ | Δ |
| Example 27 | 21 | 6 | ○ | × |
| Example 28 | 18 | 4 | Δ | × |
| Example 29 | 18 | 8 | ○ | ○ |
| Example 30 | 18 | 5 | ⊚ | ○ |
| Example 31 | 18 | 4 | Δ | ○ |
| Example 32 | 21 | 15 | ⊚ | ○ |
| Comparative Example 1 | 20 | 3 | × | ○ |
| Comparative Example 2 | 20 | 4 | × | ○ |
| Comparative Example 3 | 21 | 4 | × | ○ |
| Comparative Example 4 | 19 | 4 | × | ○ |

As is apparent from Table 3, the adhesive composition of each example can provide sterilization resistance against the sterilization processing and can appropriately maintain the adhesive strength or appearance of the adhesive layer even when the sterilization processing is repeated.

In particular, in the case of Example 32 using the plate-like alumina, the spherical alumina and the amorphous shape alumina as the filler, the adhesive strength after the sterilization processing was increased.

In the adhesive composition of Comparative examples 1 to 4 that did not contain alumina, the sterilization resistance was degraded, and the adhesive strength was generally degraded in comparison with the examples. In addition, appearance of the adhesive layer after the sterilization processing was remarkably degraded in comparison with the examples.

### [Industrial Applicability]

According to the adhesive composition used in a medical instrument and the endoscope device, good sterilization resistance is provided even when the sterilization processing is repeated, and the adhesive strength and visual appearance can be appropriately maintained. In addition, it is possible to provide the endoscope device in which the members adhered by the adhesive composition cannot be easily separated.

### [Description of Reference Numerals]

1: endoscope device
2: insertion unit
3: distal end section
4: bendable section
5: flexible tube
7: manipulation unit
8: universal code
10: cured material
11: resin section
12: plate-like alumina
13: spherical alumina
14: amorphous shape alumina
21: light guide fiber
22: imaging unit
22a: lens group
23: distal end hard section
24: distal end cover
25: adhesive layer
31: bendable rubber
34: spool section
36: adhesive layer
41: insulating member
42: forceps channel
43: forceps cap
45: object lens
46: illumination lens
47: object lens frame
48: partition wall
49: cured material

## Claims

1. An adhesive composition used in a medical instrument comprising:
a main agent that includes one or more kinds of epoxy resin selected from the group consisting of bisphenol A-type epoxy resins, bisphenol F-type epoxy resins, and phenol novolac type epoxy resins;
a curing agent comprising meta xylylene diamine and/or a derivative thereof;
acrylic rubber; and
a filler which contains alumina.

2. The adhesive composition used in a medical instrument according to claim 1, wherein
the filler includes at least plate-like alumina having an aspect ratio of 2 to 99.

3. The adhesive composition used in a medical instrument according to claim 2, wherein
the filler further includes spherical alumina having an average particle diameter of 1 to 100 µm, and amorphous shape alumina having an average particle diameter of 1 to 500 nm.

4. The adhesive composition used in a medical instrument according to claim 1, wherein
the filler includes one or more kind of alumina selected from the group consisting of plate-like alumina having an aspect ratio of 2 to 99, spherical alumina having an average particle diameter of 1 to 100 µm, and amorphous shape alumina having an average particle diameter of 1 to 500 nm, and
when a total content of the main agent, the curing agent and the acrylic rubber is 100 parts by mass,
a content of the plate-like alumina is 10 to 150 parts by mass when the filler contains the plate-like alumina,
a content of the spherical alumina is 10 to 150 parts by mass when the filler contains the spherical alumina, and
a content of the amorphous shape alumina is 1 to 20 parts by mass when the filler contains the amorphous shape alumina,
and wherein when the filler contains all of the plate-like alumina, the spherical alumina and the amorphous shape alumina, a sum of a content of the plate-like alumina, the spherical alumina and the amorphous shape alumina is 10 to 150 weight parts by mass.

5. An endoscope device assembled by using the adhesive composition according to any one of claims 1 to 4.

## Patentansprüche

1. Haftzusammensetzung, die in einem medizinischen Instrument verwendet wird, aufweisend:
ein Hauptmittel, das eine oder mehrere Arten von Eopxydharz beinhaltet, das aus der Gruppe ausgewählt wird, die aus Epoxydharzen des Typs Bisphenol-A, Epoxydharzen des Typs Bisphenol-F und Eopxydharzen des Typs Phenol-Novolak besteht;
ein Aushärtemittel, das Metaxylylendiamin und/oder ein Derivat desselben aufweist;
Acrylkautschuk; und
einen Füller, der Tonerde enthält.

2. Haftzusammensetzung, die in einem medizinischen Instrument verwendet wird, nach Anspruch 1, wobei der Füller zumindest plattenförmige Tonerde mit einem Streckungsgrad von 2 zu 99 beinhaltet.

3. Haftzusammensetzung, die in einem medizinischen Instrument verwendet wird, nach Anspruch 2, wobei der Füller ferner sphärische Tonerde, die einen durchschnittlichen Partikeldurchmesser von 1 bis 100 µm aufweist, und amorph geformte Tonerde, die einen durchschnittlichen Partikeldurchmesser von 1 bis 500 nm aufweist, beinhaltet.

4. Haftzusammensetzung, die in einem medizinischen Instrument verwendet wird, nach Anspruch 1, wobei
der Füller eine oder mehrere Arten von Tonerde beinhaltet, die aus der Gruppe ausgewählt wird, die aus plattenförmiger Tonerde, die einen Streckungsgrad von 2 zu 99 hat, sphärischer Tonerde, die einen durchschnittlichen Partikeldurchmesser von 1 bis 100 µm hat, und amorph geformter Tonerde, die einen durchschnittlichen Partikeldurchmesser von 1 bis 500 nm hat, besteht, und
wenn ein Gesamtinhalt des Hauptmittels, des Aushärtemittels und des Acrylkautschuks 100 Massenteile beträgt,
ein Inhalt der plattenförmigen Tonerde zwischen 10 und 150 Masseteile liegt, wenn der Füller die plattenförmige Tonerde beinhaltet,
ein Inhalt der sphärischen Tonerde zwischen 10 und 150 Masseteile liegt, wenn der Füller die sphärische Tonerde beinhaltet, und
ein Inhalt der amorph geformten Tonerde zwischen 1 und 20 Masseteile liegt, wenn der Füller die amorph geformte Tonerde beinhaltet,
und wobei, wenn der Füller alles von der plattenförmigen Tonerde, der sphärischen Tonerde und der amorph geformten Tonerde beinhaltet, eine Summe eines Gehalts der plattenförmigen Tonerde, der sphärischen Tonerde und der amorph geformten Tonerde zwischen 10 und 150 Massenteile beträgt.

5. Endoskopvorrichtung, zusammengesetzt durch Verwendung der Haftzusammensetzung nach einem der Ansprüche 1 bis 4.

## Revendications

1. Composition adhésive utilisée dans un instrument médical comprenant :
un agent principal qui comprend un ou plusieurs types de résine époxy choisis dans le groupe consistant en les résines époxy de type bisphénol A, les résines époxy de type bisphénol F et les résines époxy de type novolaque phénolique ;
un agent de durcissement comprenant une méta xylylène diamine et/ou un dérivé de celle-ci ;
un caoutchouc acrylique ; et
une charge qui contient de l'alumine.

2. Composition adhésive utilisée dans un instrument médical selon la revendication 1, dans laquelle
la charge comprend au moins de l'alumine en paillettes ayant un facteur de forme de 2 à 99.

3. Composition adhésive utilisée dans un instrument médical selon la revendication 2, dans laquelle
la charge comprend en outre de l'alumine sphérique ayant un diamètre de particule moyen de 1 à 100 µm, et de l'alumine de forme amorphe ayant un diamètre de particule moyen de 1 à 500 nm.

4. Composition adhésive utilisée dans un instrument médical selon la revendication 1, dans laquelle
la charge comprend un ou plusieurs types d'alumine choisis dans le groupe consistant en l'alumine en paillettes ayant un rapport de forme de 2 à 99, l'alumine sphérique ayant un diamètre de particule moyen de 1 à 100 µm et l'alumine de forme amorphe ayant un diamètre de particule moyen de 1 à 500 nm, et
lorsqu'une teneur totale en l'agent principal, l'agent de durcissement et le caoutchouc acrylique est de 100 parties en masse,
une teneur en l'alumine en paillettes est de 10 à 150 parties en masse lorsque la charge contient de l'alumine en paillettes,
une teneur en l'alumine sphérique est de 10 à 150 parties en masse lorsque la charge contient de l'alumine sphérique, et
une teneur en l'alumine de forme amorphe est de 1 à 20 parties en masse lorsque la charge contient de l'alumine de forme amorphe,
et dans laquelle, lorsque la charge contient la totalité de l'alumine en paillettes, de l'alumine sphérique et de l'alumine de forme amorphe, une somme de la teneur en l'alumine en paillettes, l'alumine sphérique et l'alumine de forme amorphe est de 10 à 150 parties en poids en masse.

5. Dispositif d'endoscope assemblé en utilisant la composition adhésive selon l'une quelconque des revendications 1 à 4.
